# FASCICULE DE BREVET EUROPEEN

(11) **EP 0 832 886 B1**
(45) Date de publication et mention de la délivrance du brevet: **13.11.2002**
(21) Numéro de dépôt: 97402202.2
(22) Date de dépôt: 23.09.1997
(51) Int. Cl.: C07D 311/30, C07D 405/12, A61K 31/35

(54) **Dérivés de flavones, leur procédé de préparation et les compositions pharmaceutiques qui les contiennent**
Flavone-Derivate, Verfahren zu ihrer Herstellung und diese enthaltende pharmazeutische Zusammenstellungen
Flavon derivatives, process for their preparation, and pharmaceutical compositions containing them

(30) Priorité: 27.09.1996 FR 9611808
(43) Date de publication de la demande: 01.04.1998
(73) Titulaire: LES LABORATOIRES SERVIER, 92200 Neuilly sur Seine (FR)
(72) Inventeur: Dhainaut, Alain, 78400 Chatou (FR); Lewin, Guy, 92500 Rueil Malmaison (FR); Canet, Emmanuel, 75014 Paris (FR); Lonchampt, Michel, 94150 Chevilly la rue (FR); Rolland, Yves, 92170 Vanves (FR)

(56) Documents cités:
- EP-A- 0 319 412
- EP-A- 0 564 350
- EP-A- 0 803 503
- CHEMICAL ABSTRACTS, vol. 108, no. 26, 1988 Columbus, Ohio, US; abstract no. 131350k, V.AHLUWALIA ET AL.: "N1-(7-FLAVONYLOXY)ACETYL-N4-ARYLTHIOSEMIC ARBAZIDES" page 709; colonne 1; XP002031572 & INDIAN J. CHEM.SECT. B, vol. 26B, no. 4, 1987, DELHI, pages 381-383,

## Description

La présente invention a pour objet de nouveaux dérivés de flavones, leur procédé de préparation et les compositions pharmaceutiques qui les contiennent.

Ces nouveaux dérivés de flavones sont des inhibiteurs de phosphodiestérases du groupe 4 (PDE4) et de ce fait présentent des applications thérapeutiques tout à fait intéressantes.

En effet, les fonctions de la plupart des tissus organiques sont modulées par des substances endogènes (hormones, neurotransmetteurs, autacoïdes) ou exogènes. Pour certaines de ces substances, l'effet biologique est relayé au niveau intracellulaire par des effecteurs enzymatiques tels que l'adénylate cyclase ou la guanylate cyclase. La stimulation de ces enzymes responsables de la synthèse de nucléotides cycliques tels que l'adenosine-3',5'-monophosphate cyclique (AMPc) et la guanosine-3',5'-monophosphate cyclique (GMPc) entraîne une élévation du taux intracellulaire de ces seconds messagers impliqués dans la régulation de nombreuses fonctions biologiques (E. W. Sutherland et T. W. Rall, *Pharmacol. Rev*., *12*, (1960), 265).

La dégradation des nucléotides cycliques est assurée par une famille d'enzymes, appelées phosphodiestérases (PDE), classées actuellement en 7 groupes. La reconnaissance d'isoformes différentes à l'intérieur de chacun de ces groupes, et de la distribution tissu- ou cellule-spécifique de certaines isoformes, a stimulé la recherche d'inhibiteurs de plus en plus spécifiques de tel ou tel type d'isoenzyme (J. A. Beavo, *Physiological Rev., 75* (4), (1995), 725-749). Parmi les différentes familles de PDE, la PDE4 a été identifiée dans de très nombreux tissus ou cellules comme le cerveau, le coeur, l'endothélium vasculaire, le muscle lisse vasculaire et trachéobronchique et les cellules hématopoïétiques. L'inhibition des phosphodiestérases ralentit l'hydrolyse des nucléotides cycliques et entraîne une augmentation de la teneur en AMPc et/ou GMPc.

Les inhibiteurs de PDE4, responsables d'une augmentation des taux d'AMPc, possèdent des activités anti-inflammatoires et des effets relaxants sur le muscle lisse trachéobronchique d'où leur intérêt thérapeutique dans le domaine de la pathologie respiratoire ou des pathologies associées à un processus inflammatoire (M. N. Palfreyman, *Drugs of the Future, 20* (8), (1995), 793-804 ; J. P. Barnes, *Eur. Respir. J., 8*, (1995), 457-462 S.B. Christensen et T. J. Torphy, *Annual Reports in Médicinal Chemistry, 29,* (1994), 185-194, Academic Press).

Les composés possédant une structure flavone sont très largement décrits dans la littérature. Certains flavonoïdes sont connus comme bactéricides ou antifongiques (V. K. Ahluwalia et al., *Indian J. Chem., Sect. B*, 26B, (1987), 381-383). D'autres flavonoïdes (EP-B-319 412 ; EP-A-319 412 ; EP-A-564 350) sont diversement substitués notamment par des radicaux pipérazinyle et/ou des résidus de sucres comme le β-glucose ou le rutinose. Ces derniers sont utiles dans le traitement des affections vasculaires (insuffisance veineuse, oedème des membres inférieurs, maladie hémorroïdaire).

D'autre part, les travaux de U.R. Kuppusamy et coll. (*Biochem. Pharmacol.*, *44*(7), (1992), 1307-1315) et de Yohko Sakamoto et coll. (*Bull. Chem. Soc. Jpn., 62*(8), (1989), 2450-2454) montrent l'activité de certains flavonoïdes sur la phosphodiestérase de l'AMPc. Les flavonoïdes testés sont par exemple la quercétine, la lutéoline, la scutellaréine, la phlorétine ou encore la génistéine.

Dans le but d'obtenir des inhibiteurs sélectifs de PDE4 plus puissants et plus actifs, la demanderesse a découvert de nouveaux dérivés de flavone de structure tout à fait originale et dotés d'activités pharmacologiques très intéressantes dans le domaine de l'inhibition des phosphodiestérases du groupe 4.

Plus particulièrement, la présente invention concerne les composés de formule générale (I) : 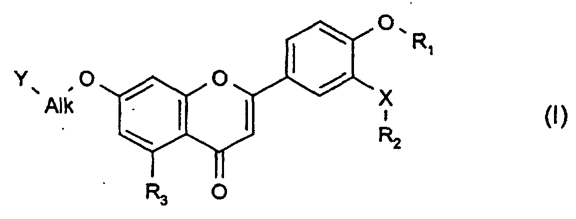 dans laquelle
- R₁ représente un radical alkyle,
- R₂ est choisi parmi :
   - un radical hydrocarboné cyclique contenant de 3 à 9 atomes de carbone, contenant éventuellement une ou plusieurs doubles liaisons intracycliques, et éventuellement substitué par un ou plusieurs atomes d'halogène, radicaux alkyle, alkoxy et/ou hydroxy,
   - un radical hydrocarboné polycyclique contenant de 6 à 15 atomes de carbone, contenant éventuellement une ou plusieurs doubles liaisons et éventuellement substitué par un ou plusieurs atomes d'halogène, radicaux alkyle, alkoxy et/ou hydroxy,
   - un radical hydrocarboné linéaire ou ramifié contenant de 1 à 13 atomes de carbone, éventuellement contenant une ou plusieurs insaturations sous forme de doubles et/ou triples liaisons -et éventuellement substitué par un ou plusieurs atomes d'halogène, radicaux hydroxy, alkoxy, aryle, hétéroaryle, radicaux hydrocarbonés cycliques définis précédemment et/ou radicaux hydrocarbonés polycycliques définis précédemment,
- R₃ est choisi parmi l'hydrogène et le radical hydroxy,
- Alk représente un radical alkylène linéaire ou ramifié comportant de 1 à 6 atomes de carbone,
- X est choisi parmi l'oxygène, le radical -CR'= et le radical -CHR'-,
- R' est choisi parmi l'hydrogène et un radical alkyle, et
- Y est choisi parmi les radicaux : 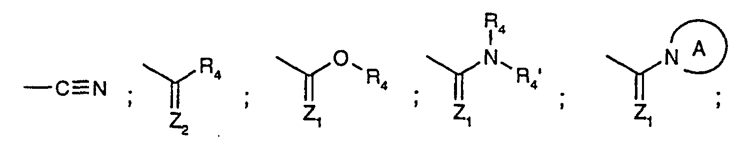 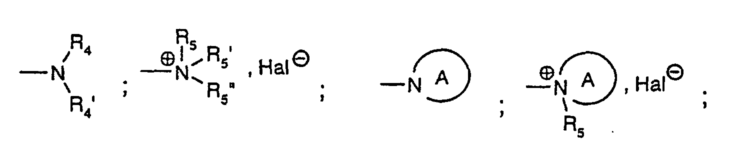 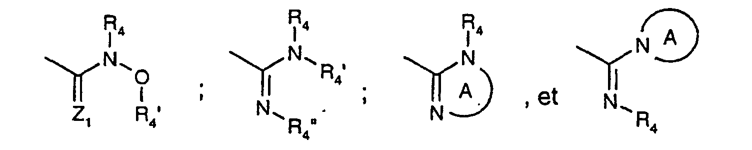 dans lesquels
   - Z₁ représente l'oxygène ou le soufre,
   - Z₂ représente l'oxygène, le soufre, un radical =N-OR₄ ou un radical 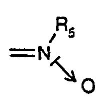
   - R₄, R₄' et R₄", identiques ou différents sont choisis indépendamment les uns des autres parmi l'hydrogène, un radical alkyle éventuellement substitué par un radical aryle ou hétéroaryle, un radical aryle, un radical hétéroaryle et un radical cycloalkyle,
   - R₅, R₅', R₅", identiques ou différents, sont choisis indépendamment les uns des autres parmi un radical alkyle éventuellement substitué par un radical aryle ou un radical hétéroaryle, un radical aryle, un radical hétéroaryle, et un radical cycloalkyle,
   - A représente un radical mono- ou bi-cyclique saturé ou insaturé comportant un total de 5 à 10 atomes (parmi lesquels, au total, 1, 2 ou 3 d'entre eux peuvent éventuellement représenter un hétéroatome choisi parmi l'oxygène, le soufre et/ou l'azote) et éventuellement substitué par un ou plusieurs atomes d'halogène, radicaux alkyle et/ou alkoxy, et
   - Hal représente un atome d'halogène,
- ou bien les radicaux -Alk-Y forment ensemble un radical choisi parmi : 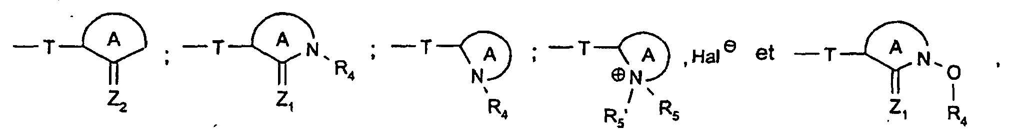 dans lesquels A, R₄, R₅, R₅', Z₁, Z₂ et Hal sont tels que définis précédemment, et T représente soit une liaison soit un radical alkylène linéaire ou ramifié comportant de 1 à 6 atomes de carbone,
à la condition que lorsque R₂ représente un radical hydrocarboné linéaire ou ramifié tel que défini plus haut, Y soit différent du radical 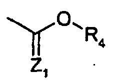 étant entendu que sauf précisions contraires,
- le terme "alkyle" représente un radical alkyle contenant de 1 à 6 atomes de carbone en chaîne droite ou ramifiée et éventuellement substitué par un ou plusieurs atomes d'halogène, radicaux hydroxy et/ou alkoxy,
- le terme "alkoxy" représente un radical alkoxy contenant de 1 à 6 atomes de carbone en chaîne droite ou ramifiée et éventuellement substitué par un ou plusieurs atomes d'halogène et/ou radicaux hydroxy,
- le terme "aryle" représente un radical phényle ou naphtyle, éventuellement substitué par un ou plusieurs atomes d'halogène, radicaux alkyle, hydroxy et/ou alkoxy,
- le terme "hétéroaryle" représente un radical choisi parmi furyle, thiényle, thiazolyle, isothiazolyle, oxazolyle, isoxazolyle, pyrrolyle, imidazolyle, pyrazolyle, pyridyle, pyrazinyle, pyrimidyle, pyridazinyle, indolyle, quinolyle et isoquinolyle, quinazolinyle, éventuellement substitué par un ou plusieurs atomes d'halogène, radicaux alkyle, hydroxy et/ou alkoxy,
- le terme "atome d'halogène" représente un atome de fluor, de chlore, de brome ou d'iode,
- le terme "cycloalkyle" représente un radical hydrocarboné cyclique et saturé contenant de 3 à 8 atomes de carbone et éventuellement substitué par un ou plusieurs atomes d'halogène, radicaux alkyle, hydroxy et/ou alkoxy,
leurs éventuels isomères optiques et/ou géométriques sous forme pure ou en mélange, et leurs éventuels sels d'addition à un acide ou à une base, pharmaceutiquement acceptables.

Parmi les acides que l'on peut utiliser pour former un sel d'addition pharmaceutiquement acceptable avec les composés de l'invention, on peut citer, à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

Parmi les bases que l'on peut utiliser pour former un sel d'addition pharmaceutiquement acceptable avec les composés de l'invention, on peut citer à titre d'exemples et de façon non limitative, les hydroxydes de sodium, de potassium, de calcium, ou d'aluminium, les carbonates de métaux alcalins ou alcalino-terreux, et les bases organiques comme la triéthylamine, la benzylamine, la diéthanolamine, la tert-butylamine, la dicyclohexylamine, et l'arginine.

La présente invention a également pour objet le procédé de préparation des composés de formule (I) caractérisé en ce que l'on fait réagir, selon le mode opératoire décrit par M. Cushman et coll. (*Tetrahedron Lett., 31*(45), (1990), 6497-6500), le composé de formule (II) : 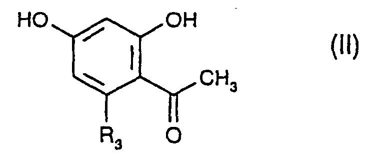 dans laquelle R₃ est tel que défini précédemment,
avec le composé de formule (III) : 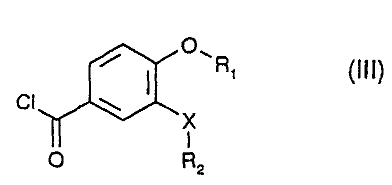 dans laquelle R₁, R₂ et X sont tels que définis précédemment,
en présence d'un excès de bis(triméthylsilyl)amidure de lithium afin d'obtenir le composé de formule (IV) : 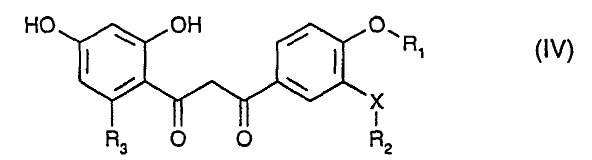 dans laquelle R₁, R₂, R₃ et X sont tels que définis précédemment,
qui est cyclisé, par chauffage dans de l'acide acétique glacial et en présence d'acide sulfurique, en composé de formule (V) : 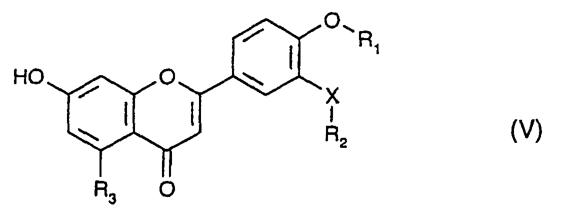 dans laquelle R₁, R₂, R₃ et X sont tels que définis précédemment,
qui est finalement couplé
- soit avec un composé de formule (VIa) :

   Y-Alk-Hal (VIa)

   dans laquelle Y et Alk sont tels que définis précédemment et Hal représente un atome d'halogène choisi parmi, chlore, brome et iode,
en présence d'hydrogénocarbonate de potassium à chaud, sous atmosphère inerte et en solvant polaire aprotique, tel que le diméthylformamide,
- soit avec un composé de formule (VIb) :

   Y-Alk-OH (VIb)

   dans laquelle Y et Alk sont tels que définis précédemment,
en présence d'azodicarboxylate d'éthyle et de triphénylphosphine (réaction de "Mitsunobu"),
afin de conduire aux composés de formule (I) qui peuvent être, le cas échéant et si on le désire
- séparés suivant une ou plusieurs méthodes de purification choisies parmi la cristallisation, la chromatographie sur gel de silice, l'extraction, la filtration et le passage sur charbon ou résine,
- séparés, sous forme pure ou sous forme de mélange, en leurs éventuels isomères optiques et/ou géométriques, selon des techniques classiques de séparation, et/ou
- transformés, par un acide ou une base, en sels pharmaceutiquement acceptables.

Les composés de formule (V) pour lesquels X représente l'oxygène, R₁ représente le radical méthyle et R₃ représente le radical hydroxy peuvent avantageusement être obtenus à partir de la diosmétine que l'on fait réagir sur le bromure de benzyle, en présence d'hydrogénocarbonate de potassium dans le diméthylformamide, pour obtenir le composé de formule (VII) : 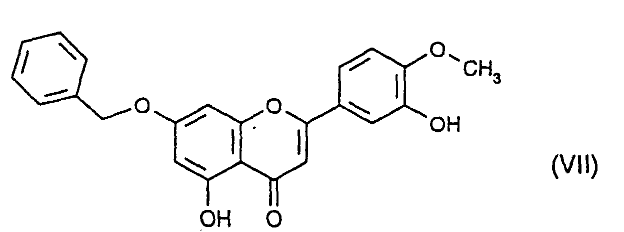 qui est ensuite traité par un halogénure de formule (VIII) :

R₂-Hal (VIII)

dans laquelle R₂ est tel que défini précédemment et Hal représente un atome d'halogène choisi parmi chlore, brome et iode,
en présence d'hydrogénocarbonate de potassium dans le diméthylformamide, conduisant au composé de formule (IX) : 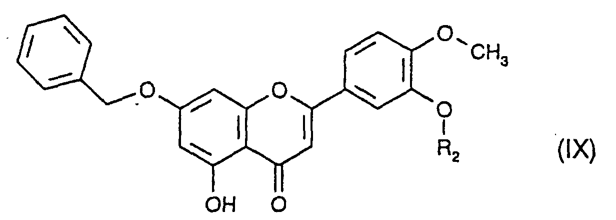 dans laquelle R₂ est tel que défini précédemment,
pour conduire après débenzylation, sous l'action d'hydrogène et en présence de palladium sur charbon, au composé de formule (X) : 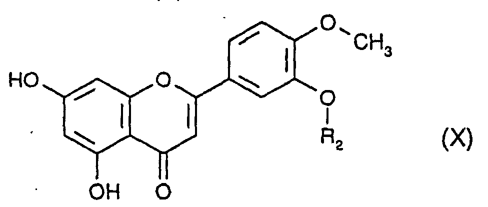 dans laquelle R₂ est tel que défini précédemment,
cas particulier des composés de formule (V) pour lesquels X représente l'oxygène, R₁ représente le radical méthyle et R₃ représente le radical hydroxy.

Les composés de formule (I) pour lesquels Y représente les radicaux: 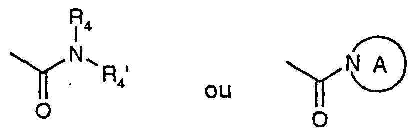 dans lesquels A, R₄ et R₄' sont tels que définis précédemment,
peuvent également être obtenus à partir des composés de formule (I) correspondants pour pour lesquels Y représente le radical 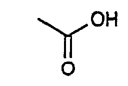 en effectuant un couplage peptidique (par exemple selon la méthode décrite par M. Bodanszky et A. Bodanszky, *The Practice of Peptide Synthesis*, Springer-Verlag, 1984) et plus particulièrement un couplage peptidique utilisant le dicyclohexylcarbodiimide (DCC) ou un de ses dérivés. La liaison amide peut également être réalisée en présence de tétrafluoroborate de 2-(1*H*-benzotriazol-1-yl)-1,1,3,3-tétraméthyluronium (TBTU), éventuellement en présence d'un activateur tel que l'hydroxybenzotriazole (HOBT) selon les méthodes décrites par M. S. Bernatowicz et coll. (*Tetrahedron Lett., 30*, (1989), 4645), A. G. Beck-Sickinger et coll. (*Pept. Res., 4*, (1991), 88), G. E. Reid et coll. (*Anal. Biochem., 200*, (1992), 301) ou encore par C. G. Fields et coll. (*Pept. Res., 4*, (1991), 95). Il peut être aussi particulièrement intéressant de former la liaison amide en présence d'anhydride propylphosphonique et de N-éthylmorpholine selon la méthode décrite par H. Wissmann et coll. (*Angew. Chem. Int. Ed., 19*, (1980), 133-134).

Les composés de formule (I) pour lesquels Y représente le radical -C(O)NR₄OR₄' dans lesquels R₄ et R₄' sont tels que définis précédemment, peuvent également être obtenus à partir des composés de formule (I) correspondants pour lesquels Y représente le radical -C(O)OR₄, dans lequel R₄ est tel que défini précédemment, en les faisant réagir avec un composé de formule NHR₄-OR₄', R₄ et R₄' étant tels que définis précédemment.

Les composés de formule (I) pour lesquels Y représente le radical 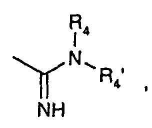 R₄ et R₄' étant tels que définis précédemment, ou le radical 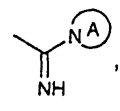 dans lequel A est tel que défini précédemment, peuvent également être obtenus à partir des composés de formule (I) correspondants pour lesquels Y représente le radical -C≡N par traitement par une amine de formule HNR₄R₄', R₄ et R₄' étant tels que définis précédemment, ou respectivement par une amine cyclique de formule 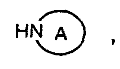 A étant tel que défini précédemment.

De manière générale, les composés de formule (I) pour lesquels Z₁ ou Z₂ représente le soufre peuvent avantageusement être obtenus à partir des composés de formule (I) correspondants pour lesquels Z₁ ou Z₂ représentent l'oxygène, par traitement par le réactif de Lawesson.

Les composés de formule (I) pour lesquels Z₂ représente le radical =N-OR₄ ou le radical 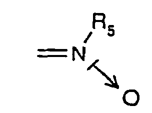 dans lesquels R₄ et R₅ sont tels que définis précédemment, sont avantageusement obtenus à partir des composés de formule (I) correspondants pour lesquels Z₂ représente l'oxygène, par action de H₂N-OR₄ ou respectivement de R₅-NHOH, R₄ et R₅ étant tels que définis précédemment.

Les composés de formule (I) dans lesquels Y contient une fonction ammonium quaternaire sont avantageusement obtenus à partir des composés de formule (I) correspondants dans lesquels Y contient une fonction amine tertiaire, que l'on traite par un halogénure de formule Hal-R₅, dans laquelle Hal représente un atome de chlore, de brome ou d'iode et R₅ est tel que défini précédemment.

Les composés de la présente invention sont des inhibiteurs très puissants des phosphodiestérases du groupe 4 et de ce fait sont particulièrement intéressants dans les applications thérapeutiques concernant l'inflammation et la relaxation bronchique et plus précisément dans l'asthme et les bronchopathies chroniques obstructives (A. J. Duplantier et coll., *Annu. Rep. Med. Chem., 29*, (1994), 73-81), (C. D. Nicholson et coll., *Pulmonary Pharmacol., 7*, (1994), 1-17), (T. J. Torphy et coll., *Drug News Perspect., 6*, (1993), 203-214), (J. A. Lowe et coll., *Drugs Future, 17*, (1992), 799-807), mais aussi dans toutes les affections telles que les rhinites (I. Raderer et coll., *Wien. Med. Wochenschr., 145*, (1995), 456-458), le syndrome de détresse respiratoire aigu (SDRA) (C. R. Turner et coll., *Circulatory Shock, 39*, (1993), 237-245), les allergies et les dermatites (J. M. Hanifin et coll., *J. lnvest. Dermatol., 105*, (1995), 84S-88S), (J. M. Hanifin, *J. Dermatol. Sci., 1*, (1990), 1-6), le psoriasis (E. Touitou et coll., *J. Pharm. Sci., 81*, (1992), 131-134), (F. Levi-schaffer et coll., *Skin Pharmacol., 4,* (1991), 286-290), l'arthrite rhumatoïde (J. M. Anaya et coll., *J. Rheumatol., 22,* (1995), 595-599), les maladies autoimmunes, (C. P. Genain et coll. *Proc. Natl. Acad. Sci., 92*, (1995), 3601-3605), les scléroses multiples (N. Sommer et coll., *Nat. Med., 1*, (1995), 244-248), les dyskinésies (T. Kitatani et coll., *Nippon Yakurigaku Zasshi, 86*, (1985), 353-358), les glomérulonéphrites (M. Hechtet et coll., *J. Leukoc. Biol., 57*, (1995), 242-249), l'ostéoarthrite et le choc septique (A. M. Badger et coll., *Circ. Shock, 44*, (1994), 188-195), (L. Sekut et coll., *Clin. Exp. Immunol., 100,* (1995), 126-132), le sida (T. F. Greten et coll., *Aids,* 9, (1995), 1137-1144), la dépression (N. A. Saccomano et coll., *J. Med. Chem., 34*, (1991), 291-298), et toute maladie neurodégénérative s'accompagnant de phénomènes inflammatoires comme les maladies d'Alzheimer, de Parkinson, de Huntington, de Down et la sclérose latérale amyotrophique (G. Z. Feuerstein et coll., *Ann. N. Y. Acad. Sci., 765*, (1995), 62-71).

Ces indications thérapeutiques ne sont pas limitatives dans la mesure où la diminution de la concentration d'AMPc cellulaire, quelqu'en soient la cause et la localisation tissulaire, peut aboutir à une dysfonction cellulaire, source de phénomènes pathologiques, et peut constituer une cible thérapeutique majeure pour les produits de l'invention.

La présente invention a également pour objet les compositions pharmaceutiques contenant les produits de formule (I) ou le cas échéant un de leurs sels d'addition à un acide ou une base, pharmaceutiquement acceptables, en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, les ampoules buvables ou injectables et les aérosols.

La posologie varie selon le sexe, l'âge et le poids du patient, la voie d'administration, la nature de l'indication thérapeutique, ou des traitements éventuellement associés et s'échelonne entre 1 mg et 5 g par 24 heures en 1 ou 2 prises.

Les exemples suivants illustrent l'invention sans toutefois la limiter en aucune façon. Les matières premières utilisées dans ces exemples sont soit commerciales soit directement accessibles à partir de modes opératoires connus de l'homme du métier.

### Préparation A : 3'-O-Cyclopentyldiosmétine

### Etape a : 7-O-Benzyldiosmétine

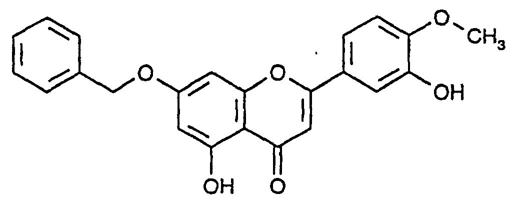

A 21 g (70 mmol) de diosmétine dans 200 ml de diméthylformamide sont ajoutés 7 g (70 mmol) d'hydrogénocarbonate de potassium. Le milieu réactionnel est agité à 110-120°C sous azote pendant 5 minutes. Sont ajoutés ensuite 12,5 ml (105 mmol) de bromure de benzyle et la réaction est poursuivie dans les mêmes conditions pendant 2,5 heures. Le milieu réactionnel est alors filtré sur verre fritté et évaporé à sec. Le résidu est repris par 300 ml de tétrahydrofurane puis 50 ml d'éthanol et le mélange est chauffé à reflux environ 15 minutes puis filtré à chaud. Le filtrat est débarrassé du tétrahydrofurane par additions successives de 250 ml d'éthanol et concentration jusqu'au début d'une cristallisation nette. Après une nuit à température ambiante les cristaux sont filtrés. 12 g du produit attendu sont obtenus.
Rendement : 44%
Point de fusion : 208-212°C

### Etape b : 7 -O-Benzyl-3' -O-cyclopentyldiosmétine

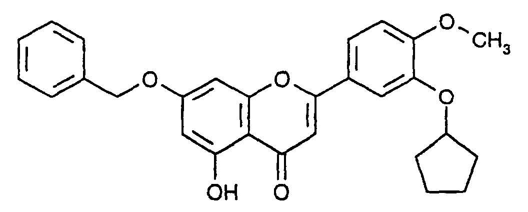

A 11,7 g (30 mmol) de 7-O-benzyldiosmétine obtenus à l'étape précédente, dans 150 ml de diméthylformamide sont ajoutés 12 g (120 mmol) d'hydrogénocarbonate de potassium. Le milieu réactionnel est agité à 110-120°C sous azote pendant 5 minutes. Sont alors ajoutés 4,3 ml (39 mmol) de bromure de cyclopentyle et la réaction est poursuivie dans les mêmes conditions pendant 2 heures. Deux nouveaux ajouts de 1,8 g d'hydrogénocarbonate de potassium et de 0,64 ml de bromure de cyclopentyle sont ensuite effectués à une heure d'intervalle. Après encore une heure de réaction, le milieu réactionnel est repris par 1,5 I d'eau et extrait au dichlorométhane. La phase organique est concentrée et le résidu sec de réaction est solubilisé dans le dichlorométhane qui est éliminé par ajouts successifs de méthanol jusqu'à cristallisation. Une recristallisation est effectuée dans les mêmes conditions pour fournir 9,73 g du composé attendu.
Rendement : 71%
Point de fusion : 208-209°C

### Etape c : 3'-O-Cyclopentyldiosmétine

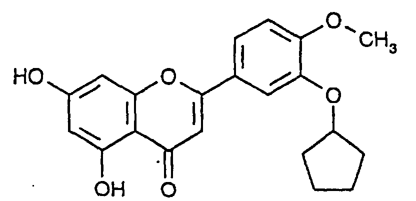

Dans 300 ml de diméthylformamide sont solubilisés à chaud 8,25 g (18 mmol) du composé obtenu à l'étape précédente. A cette solution encore tiède sont ajoutés 900 mg de palladium sur charbon à 10%. L'ensemble est soumis à hydrogénation à température ambiante et pression atmosphérique pendant 2,5 heures, filtré et évaporé à sec. Le résidu sec est alors solubilisé dans un mélange de dichlorométhane/méthanol puis dilué avec du méthanol et enfin concentré pour éliminer le dichlorométhane. 4,96 g du composé cristallisé attendu sont obtenus.
Rendement : 75%
Point de fusion : 200-201°C

### Préparation B : 3'-O-Cyclopentyl-5-désoxydiosmétine

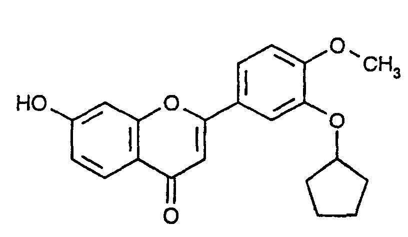

Une solution de bis-(triméthylsilyl)amidure de lithium dans le tétrahydrofurane (1M, 80 ml, 80 mmol) est ajoutée à une solution de 3,04 g (20 mmol) de 2,4-dihydroxy-acétophénone dans le tétrahydrofurane, sous argon, à -78°C en 15 minutes. Le mélange réactionnel est agité 1 heure à -78°C, deux heures à -10°C, refroidi à nouveau à -78°C, puis est additionné d'une solution de 5,09 g (20 mmol) de chlorure de 3-cyclopentyloxy-4-méthoxybenzoyle dans 15 ml de tétrahydrofurane. L'agitation est maintenue pendant une demi-heure à -78°C puis 4 heures, en laissant le milieu réactionnel revenir à température ambiante.

Après hydrolyse, extraction et séchage de l'intermédiaire dicétonique, celui-ci est traité par 100 ml d'acide acétique glacial et 0,5 ml d'acide sulfurique, à une température de 100°C pendant 1 heure. Le milieu réactionnel est concentré au quart de son volume initial et dilué dans 500 ml d'eau glacée. Le précipité est filtré et lavé à l'eau puis séché.
Rendement : 68%
Point de fusion : 228-229°C

### Préparation C : 3'-O-(5-Phénylpent-2-yl)diosmétine

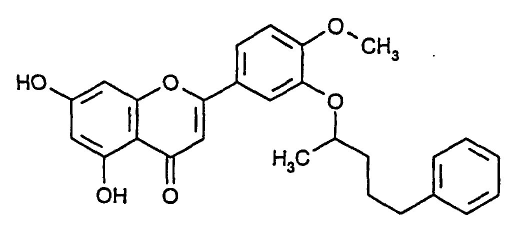

En procédant comme décrit dans la préparation A, en remplaçant le bromure de cyclopentyle par le 2-chloro-5-phénylpentane, le composé attendu est obtenu.

### EXEMPLE 1 : 7-O-(2-Oxopropyl)-3'-O-cyclopentyldiosmétine

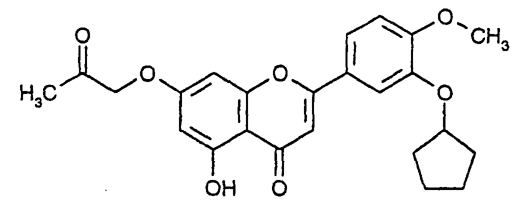

A un mélange contenant 3,7 g de 3'-O-cyclopentyldiosmétine obtenue à la préparation A et 1,3 g d'hydrogénocarbonate de potassium dans 50 ml de diméthylformamide est ajouté 0,92 g de chloropropan-2-one. Le mélange est agité sous atmosphère inerte à 120°C pendant 3 heures, puis refroidi à température ambiante et dilué avec 50 ml de dichlorométhane. Les insolubles sont filtrés et le filtrat concentré à sec. Le résidu obtenu est repris dans le méthanol. Le produit attendu cristallise, puis est filtré et séché sous vide.
Point de fusion : 170-171°C

### EXEMPLE 2 : 7-O-Ethoxycarbonylméthyl-3'-O-cyclopentyldiosmétine

A un mélange contenant 3,7 g de 3'-O-cyclopentyldiosmétine obtenue à la préparation A, 2,6 g de triphénylphosphine et 1,1 g de glycolate d'éthyle dans 50 ml de tétrahydrofurane sont ajoutés 2,2 g d'azodicarboxylate d'éthyle. Ce mélange est agité 24 heures à température ambiante sous atmosphère inerte. Le milieu réactionnel est alors concentré et purifié par cbromatographie flash (éluant : dichlorométhane/méthanol, 99:1). Le composé attendu est recristallisé dans le méthanol.
Point de fusion : 146-147°C

En opérant selon le mode opératoire décrit dans l'exemple 1, en remplaçant la 1-chloropropan-2-one par le dérivé halogéné approprié, les composés suivants sont obtenus.

### EXEMPLE 3 : 7-O-Cyanométhyl-3'-O-cyclopentyldiosmétine

Point de fusion : 189-190°C

### EXEMPLE 4 : 7-O-t-Butoxycarbonylméthyl-3'-O-cyclopentyldiosmétine

Point de fusion : 160-161°C

### EXEMPLE 5 : 7-O-(3-Ethoxycarbonylpropyl)-3'-O-cyclopentyldiosmétine

Point de fusion : 78-79°C

### EXEMPLE 6 : 7-O-Benzoylméthyl-3'-O-cyclopentyldiosmétine

Point de fusion : 185-186°C

### EXEMPLE 7 : 7-O-(4-Oxopentyl)-3'-O-cyclopentyldiosmétine

Point de fusion : 109-110°C

### EXEMPLE 8 : 7-O-(3-Oxobut-2-yl)-3'-O-cyclopentyldiosmétine

Point de fusion : 120-121°C

### EXEMPLE 9 : 7-O-(2-Oxocyclopentyl)-3'-O-cyclopentyldiosmétine

Point de fusion : 190-191°C

### EXEMPLE 10 : 7-O-(2-Oxobutyl)-3'-O-cyclopentyldiosmétine

Point de fusion : 192-193°C

### EXEMPLE 11 : 7-O-(Carboxyméthyl)-3'-O-cyclopentyldiosmétine

| Analyse élémentaire : (formule brute : C₂₃H₂₂O₈ poids moléculaire : 426) | | |
|---|---|---|
| | **C** | **H** |
| % trouvé | 64,71 | 5,46 |
| % calculé | 64,78 | 5,20 |

### EXEMPLE 12 : 7-O-(N-Méthoxy-N-méthylaminocarbonylméthyl)-3'-O-cyclopentyldiosmétine

Point de fusion : 194-195°C

### EXEMPLE 13 : 7-O-Morpholinocarbonylméthyl-3'-O-cyclopentyldiosmétine

En opérant un couplage peptidique entre l'acide obtenu à l'exemple 11 et la morpholine, le composé attendu est obtenu.
Point de fusion : 128-130°C

### EXEMPLE 14 : 7-O-(N,N-Diéthylaminocarbonylméthyl)-3'-O-cyclopentyldiosmétine

En opérant comme décrit dans l'exemple précédent en remplaçant la morpholine par la diéthylamine, le composé attendu est obtenu.
Point de fusion : 79-80°C

### EXEMPLE 15 : 7-O-(N,N-Diéthylaminothiocarbonylméthyl)-3'-O-cyclopentyldiosmétine

L'amide obtenu à l'exemple 14 est traité par le réactif de Lawesson afin de conduire au composé attendu.
Point de fusion : 85-87°C

### EXEMPLE 16 : 7-O-[(4-Méthylpipérazin-1-yl)carbonylméthyl]-3'-O-cyclopentyldiosmétine

En remplaçant dans l'exemple 13 la morpholine par la N-méthylpipérazine, le composé attendu est obtenu.
Point de fusion : 144-145°C

De la même façon, en utilisant les amines appropriées, les composés des exemples 17 à 19 suivants sont obtenus.

### EXEMPLE 17 : 7-O-(N-Méthylaminocarbonylméthyl)-3'-O-cyclopentyldiosmétine

Point de fusion : 209-210°C

### EXEMPLE 18 : 7-O-Aminocarbonylméthyl-3'-O-cyclopentyldiosmétine

Point de fusion : 215-217°C

### EXEMPLE 19 : 7-O-(N,N-Diméthylaminocarbonylméthyl)-3'-O-cyclopentyldiosmétine

Point de fusion : 187-188°C

### EXEMPLE 20 : 7-O-(N-Hydroxy-aminocarbonylméthyl)-3'-O-cyclopentyldiosmétine

L'ester obtenu à l'exemple 2 est traité par l'hydroxylamine pour conduire à l'acide hydroxamique attendu.
Point de fusion : 189-191°C

En procédant selon le mode opératoire décrit à l'exemple 2, en remplaçant le glycolate d'éthyle par l'amino-alcool approprié, les composés suivants sont obtenus.

### EXEMPLE 21 : 7-O-(Morpholino-éthyl)-3'-O-cyclopentyldiosmétine

Point de fusion : 145-146°C

### EXEMPLE 22 : 7-O-(4-Méthylpipérazin-1-yléthyl)-3'-O-cyclopentyldiosmétine

### EXEMPLE 23 : 7-O-(N,N-Diméthylamino-éthyl)-3'-O-cyclopentyldiosmétine

Point de fusion : 154-155°C

### EXEMPLE 24 : Iodure de 7-O-(N,N,N-Triméthylammonio-éthyl)-3'-O-cyclopentyldiosmétine

Composé obtenu par traitement du composé de l'exemple 23 par l'iodure de méthyle. Point de fusion : > 166°C (décomposition)

### EXEMPLE 25 : 7-O-(N-Méthylpipéridin-2-ylméthyl)-3'-O-cyclopentyldiosmétine

Composé obtenu en procédant selon le mode opératoire décrit à l'exemple 2, en remplaçant le glycolate d'éthyle par la 2-hydroxy-N-méthylpipéridine.

### EXEMPLE 26 : 7-O-(N-Ethoxyaminocarbonylméthyl)-3'-O-cyclopentyldiosmétine

Le composé obtenu à l'exemple 3, traité par la O-éthylhydroxylamine, fournit après hydrolyse, le composé attendu.

### EXEMPLE 27 : 7-O-(N-Méthylcarbamimidoylméthyl)-3'-O-cyclopentyldiosmétine

Le composé obtenu à l'exemple 3, traité par la méthylamine, conduit à l'amidine attendue.

En opérant comme décrit à l'exemple 27, en remplaçant la méthylamine par l'amine appropriée, les composés suivants sont obtenus.

### EXEMPLE 28 : 7-O-(N¹,N¹-Diéthylcarbamimidométhyl)-3'-O-cyclopentyldiosmétine

### EXEMPLE 29 : 7-O-(2-Morpholino-2-imino-éthyl)-3'-O-cyclopentyldiosmétine

### EXEMPLE 30 : 7-O-(2-Hydroxyiminopropyl)-3'-O-cyclopentyldiosmétine

Le traitement du composé obtenu à l'exemple 1 par l'hydroxylamine fournit le composé attendu.
Point de fusion : 190-193°C

### EXEMPLE 31 : 7-O-(2-Ethoxy-iminocyclopentyl)-3'-O-cyclopentyldiosmétine

Le traitement du composé obtenu à l'exemple 9 par la O-éthylhydroxylamine conduit au composé attendu.

### EXEMPLE 32 : N-Oxyde de 7-O-[2-(N-éthylimino)propyl]-3'-O-cyclopentyldiosmétine

Le composé obtenu à l'exemple 1 est traité par la N-éthylhydroxylamine pour conduire au composé attendu.

### EXEMPLE 33 : N-Oxyde de 7-O-[2-(N-éthylimino)cyclopentyl]-3'-O-cyclopentyldiosmétine

En procédant comme décrit dans l'exemple précédent mais en opérant à partir du composé de l'exemple 9, le composé attendu est obtenu.

### EXEMPLE 34 : 7-O-Ethoxycarbonylméthyl-3'-O-cyclopentyl-5-désoxydiosmétine

En procédant selon le mode opératoire décrit à l'exemple 2, à partir de la 5-désoxydiosmétine obtenue dans la préparation B, le composé attendu est obtenu.
Point de fusion : 120-121°C

De la même façon, en procédant selon les modes opératoires des exemples 1 et 3 à 24 précédents et en utilisant la 5-désoxydiosmétine à la place de la diosmétine, les composés suivants sont obtenus.

### EXEMPLE 35 : 7-O-(2-Oxopropyl)-3'-O-cyclopentyl-5-désoxydiosmétine

### EXEMPLE 36 : 7-O-Cyanométhyl-3'-O-cyclopentyl-5-désoxydiosmétine

### EXEMPLE 37 : 7-O-t-Butoxycarbonylméthyl-3'-O-cyclopentyl-5-désoxydiosmétine

### EXEMPLE 38 : 7-O-(3-Ethoxycarbonylpropyl)-3'-O-cyclopentyl-5-désoxydiosmétine

### EXEMPLE 39 : 7-O-Benzoylméthyl-3'-O-cyclopentyl-5-désoxydiosmétine

### EXEMPLE 40 : 7-O-(4-Oxopentyl)-3'-O-cyclopentyl-5-désoxydiosmétine

### EXEMPLE 41 : 7-O-(3-Oxobut-2-yl)-3'-O-cyclopentyl-5-désoxydiosmétine

### EXEMPLE 42 : 7-O-(2-Oxocyclopentyl)-3'-O-cyclopentyl-5-désoxydiosmétine

### EXEMPLE 43 : 7-O-(2-Oxobutyl)-3'-O-cyclopentyl-5-désoxydiosmétine

### EXEMPLE 44 : 7-O-(Carboxyméthyl)-3'-O-cyclopentyl-5-désoxydiosmétine

### EXEMPLE 45 : 7-O-(N-Méthoxy-N-méthylaminocarbonylméthyl)-3'-O-cyclopentyl-5-désoxydiosmétine

### EXEMPLE 46 : 7-O-Morpholinocarbonylméthyl-3'-O-cyclopentyl-5-désoxydiosmétine

### EXEMPLE 47 : 7-O-(N,N-Diéthylaminocarbonylméthyl)-3'-O-cyclopentyl-5-désoxydiosmétine

### EXEMPLE 48 : 7-O-(N,N-Diéthylaminothiocarbonylméthyl)-3'-O-cyclopentyl-5-désoxydiosmétine

### EXEMPLE 49 : 7-O-[(4-Méthylpipérazin-1-yl)carbonylméthyl]-3'-O-cyclopentyl-5-désoxydiosmétine

### EXEMPLE 50 : 7-O-(N-Méthylaminocarbonylméthyl)-3'-O-cyclopentyl-5-désoxydiosmétine

### EXEMPLE 51 : 7-O-Aminocarbonylméthyl-3'-O-cyclopentyl-5-désoxydiosmétine

### EXEMPLE 52 : 7-O-(N,N-Diméthylaminocarbonylméthyl)-3'-O-cyclopentyl-5-désoxydiosmétine

### EXEMPLE 53 : 7-O-(N-Hydroxy-aminocarbonylméthyl)-3'-O-cyclopentyl-5-désoxydiosmétine

### EXEMPLE 54 : 7-O-(Morpholino-éthyl)-3'-O-cyclopentyl-5-désoxydiosmétine

### EXEMPLE 55 : 7-O-(4-Méthylpipérazin-1-yléthyl)-3'-O-cyclopentyl-5-désoxydiosmétine

### EXEMPLE 56 : 7-O-(N,N-Diméthylamino-éthyl)-3'-O-cyclopentyl-5-désoxydiosmétine

### EXEMPLE 57 : lodure de 7-O-(N,N,N-Triméthylammonio-éthyl)-3'-O-cyclopentyl-5-désoxydiosmétine

En procédant selon les modes opératoires décrits dans les exemples précédents, en utilisant la 3'-O-(5-phénylpent-2-yl)diosmétine obtenue à la préparation C à la place de la 3'-O-cyclopentyldiosmétine, les composés suivants sont obtenus.

### EXEMPLE 58 : 7-O-(2-Oxopropyl)-3'-O-(5-phénylpent-2-yl)diosmétine

### EXEMPLE 59 : 7-O-Ethoxycarbonylméthyl-3'-O-(5-phénylpent-2-yl)diosmétine

### EXEMPLE 60 : 7-O-Cyanométhyl-3'-O-(5-phénylpent-2-yl)diosmétine

### EXEMPLE 61 : 7-O-t-Butoxycarbonylméthyl-3'-O-(5-phénylpent-2-yl)diosmétine

### EXEMPLE 62 : 7-O-(3-Ethoxycarbonylpropyl)-3'-O-(5-phénylpent-2-yl)diosmétine

### EXEMPLE 63 : 7-O-Benzoylméthyl-3'-O-(5-phénylpent-2-yl)diosmétine

### EXEMPLE 64 : 7-O-(4-Oxopentyl)-3'-O-(5-phénylpent-2-yl)diosmétine

### EXEMPLE 65 : 7-O-(3-Oxobut-2-yl)-3'-O-(5-phénylpent-2-yl)diosmétine

### EXEMPLE 66 : 7-O-(2-Oxocyclopentyl)-3'-O-(5-phénylpent-2-yl)diosmétine

### EXEMPLE 67 : 7-O-(2-Oxobutyl)-3'-O-(5-phénylpent-2-yl)diosmétine

### EXEMPLE 68 : 7-O-(Carboxyméthyl)-3'-O-(5-phénylpent-2-yl)diosmétine

### EXEMPLE 69 : 7-O-(N-Méthoxy-N-méthylaminocarbonylméthyl)-3'-O-(5-phénylpent-2-yl)-diosmétine

### EXEMPLE 70 : 7-O-Morpholinocarbonylméthyl-3'-O-(5-phénylpent-2-yl)diosmétine

### EXEMPLE 71 : 7-O-(N,N-Diéthylaminocarbonylméthyl)-3'-O-(5-phénylpent-2-yl)diosmétine

### EXEMPLE 72 : 7-O-(N,N-Diéthylaminothiocarbonylméthyl)-3'-O-(5-phénylpent-2-yl)diosmétine

### EXEMPLE 73 : 7-O-[(4-Méthylpipérazin-1-yl)carbonylméthyl]-3'-O-(5-phénylpent-2-yl)diosmétine

### EXEMPLE 74 : 7-O-(N-Méthylaminocarbonylméthyl)-3'-O-(5-phénylpent-2-yl)diosmétine

### EXEMPLE 75 : 7-O-Aminocarbonylméthyl-3'-O-(5-phénylpent-2-yl)diosmétine

### EXEMPLE 76 : 7-O-(N,N-Diméthylaminocarbonylméthyl)-3'-O-(5-phénylpent-2-yl)diosmétine

### EXEMPLE 77 : 7-O-(N-Hydroxy-aminocarbonylméthyl)-3'-O-(5-phénylpent-2-yl)diosmétine

### EXEMPLE 78 : 7-O-(Morpholino-éthyl)-3'-O-(5-phénylpent-2-yl)diosmétine

### EXEMPLE 79 : 7-O-(4-Méthylpipérazin-1-yléthyl)-3'-O-(5-phénylpent-2-yl)diosmétine

### EXEMPLE 80 : 7-O-(N,N-Diméthylamino-éthyl)-3'-O-(5-phénylpent-2-yl)diosmétine

### EXEMPLE 81 : lodure de 7-O-(N,N,N-triméthylammonio-éthyl)-3'-O-(5-phénylpent-2-yl)-diosmétine

### EXEMPLE 82 : 7-(N,N-Diméthylaminocarbonylméthyloxy)-5-hydroxy-4'-méthoxy-3'-pentylflavone

### Etape a : 5,7-Dihydroxy-4'-méthoxy-3'-pentylflavone

En procédant comme décrit dans la préparation B, en remplaçant le chlorure de 3-cyclopentyloxy-4-méthoxybenzoyle par le chlorure de 4-méthoxy-3-pentylbenzoyle, et la 2,4-dihydroxyacétophénone par la 2,4,6-trihydroxyacétophénone, le composé attendu est obtenu.
Point de fusion : 166-168°C

### Etape b : 7-(N,N)-5-hydroxy-4'-méthoxy-3'-pentylflavone

Le traitement du composé obtenu à l'étape précédente par le N,N-diméthylchloroacétamide, selon le mode opératoire décrit à l'exemple 1, conduit au composé attendu.
Point de fusion : 112-114°C

Les composés suivants sont obtenus selon les modes opératoires décrits précédemment.

### EXEMPLE 83 : 4'-Ethoxy-5-hydroxy-3'-(pent-1-ényl)-7-(2-oxopropoxy)flavone

### EXEMPLE 84 : 3'-O-Adamantyl-7-O-(2-oxopropyl)diosmétine

### EXEMPLE 85 : 7-O-(N,N-Diméthylaminocarbonylméthyl)-3'-O-(exo-norborn-2-yl)diosmétine

Point de fusion : 156-157°C

### EXEMPLE 86 : 3'-Allyl-4'-méthoxy-5-hydroxy-7-(2-oxopropoxy)flavone

### EXEMPLE 87 : 3'-O-(2-Méthylcyclopropylméthyl)-7-O-(2-oxopropyl)-5-désoxydiosmétine

### EXEMPLE 88 : 3'-O-Cyclopentyl-7-O-(quinol-4-yléthyl)diosmétine

### EXEMPLE 89 : 3'-O-Cyclopentyl-7-O- (quinazolin -2 -ylpropyl )diosmétine

### EXEMPLE 90 : 7-O-(N,N-Diméthylaminocarbonylméthyl)-3'-O-(3-méthylbutyl)diosmétine

Point de fusion : 151-153°C

### EXEMPLE 91 : 7-O-(N,N-Diméthylaminocarbonylméthyl)-3'-O-isopropyldiosmétine

Point de fusion : 190-193°C

### EXEMPLE 92 : 3'-O-Allyl-7-O-(N,N-Diméthylaminocarbonylméthyl)diosmétine

Point de fusion : 202-204°C

### EXEMPLE 93 : 7-O-(N,N-Diméthylaminocarbonylméthyl)-3'-O-pentyldiosmétine

Point de fusion : 181-182°C

### EXEMPLE 94 : 7-O-(N-Benzyloxyaminocarbonylméthyl)-3'-O-cyclopentyldiosmétine

Point de fusion : 172-174°C

### ETUDE PHARMACOLOGIQUE

### EXEMPLE A : Mesure de l'activité PDE

Les cellules U937 sont cultivées dans un milieu de culture (RPMI) contenant 10% de sérum de veau foetal. Brièvement, les cellules sont lysées puis ensuite centrifugées (100000 g, 60 min., 4°C) et le surnageant est récupéré en vue d'une séparation par HPLC des différentes formes de PDE (C. Lugnier et V.B. Schini, *Biochem. Pharmacol., 39*, (1990), 75-84).

L'activité PDE est mesurée par l'apparition de [³H]5' AMP résultant de l'hydrolyse du [³H]AMP cyclique. La PDE et le [³H]AMP cyclique (1 µCi/ml) sont incubés 30 minutes à 30°C. La radioactivité est mesurée au moyen d'un compteur à scintillation liquide (Beckman LS 1701).

La PDE 4 est caractérisée par :
- l'hydrolyse de l'AMP cyclique,
- l'absence d'inhibition par le GMP cyclique de l'hydrolyse d'AMP cyclique, et
- l'inhibition par le rolipram, molécule de référence.

Les composés sont étudiés à deux concentrations (10⁻⁷M et 10⁻⁵M) en duplicate. Les résultats sont exprimés en % d'inhibition de l'activité phosphodiestérasique.
Les composés de la présente invention présentent une inhibition très importante de l'activité phosphodiestérasique, inhibition qui, par exemple, peut dépasser 60%, dès la concentration de 10⁻⁷M.

### EXEMPLE B : Composition pharmaceutique : comprimés

### Formule de préparation pour 1000 comprimés dosés à 1 mg de 7-O-(2-oxopropyl)-3'-O-cyclopentyldiosmétine.

| | |
|---|---|
| 7-O-(2-oxopropyl)-3'-O-cyclopentyldiosmétine | 1 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule générale (I) : 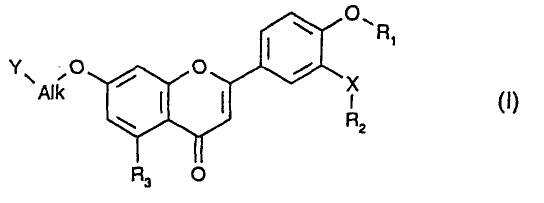 dans laquelle
- R₁ représente un radical alkyle,
- R₂ est choisi parmi:
- un radical hydrocarboné cyclique contenant de 3 à 9 atomes de carbone, contenant éventuellement une ou plusieurs doubles liaisons intracycliques, et éventuellement substitué par un ou plusieurs atomes d'halogène, radicaux alkyle, alkoxy et/ou hydroxy,
- un radical hydrocarboné polycyclique contenant de 6 à 15 atomes de carbone, contenant éventuellement une ou plusieurs doubles liaisons et éventuellement substitué par un ou plusieurs atomes d'halogène, radicaux alkyle, alkoxy et/ou hydroxy,
- un radical hydrocarboné linéaire ou ramifié contenant de 1 à 13 atomes de carbone, éventuellement contenant une ou plusieurs insaturations sous forme de doubles et/ou triples liaisons et éventuellement substitué par un ou plusieurs atomes d'halogène, radicaux hydroxy, alkoxy, aryle, hétéroaryle, radicaux hydrocarbonés cycliques définis précédemment et/ou radicaux hydrocarbonés polycycliques définis précédemment,
- R₃ est choisi parmi l'hydrogène et le radical hydroxy,
- Alk représente un radical alkylène linéaire ou ramifié comportant de 1 à 6 atomes de carbone,
- X est choisi parmi l'oxygène, le radical -CR'= et le radical -CHR'-,
- R' est choisi parmi l'hydrogène et un radical alkyle, et
- Y est choisi parmi les radicaux : 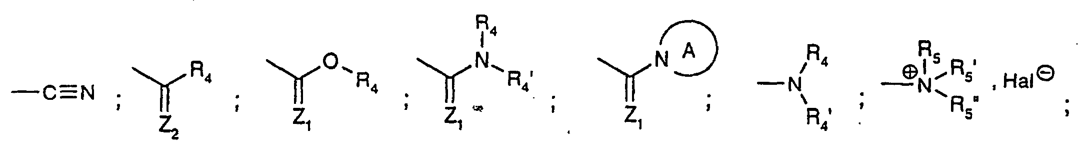 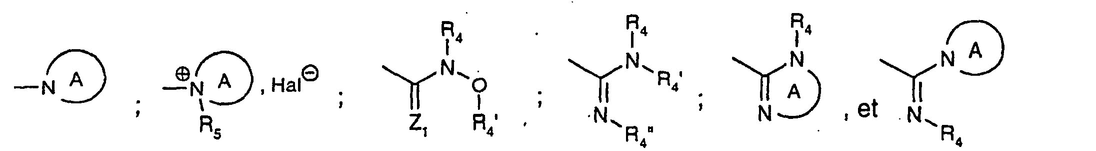 dans lesquels :
- Z₁ représente l'oxygène ou le soufre,
- Z₂ représente l'oxygène, le soufre, un radical =N-OR₄ ou un radical 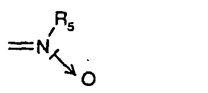
- R₄, R₄' et R₄", identiques ou différents sont choisis indépendamment les uns des autres parmi l'hydrogène, un radical alkyle éventuellement substitué par un radical aryle ou hétéroaryle, un radical aryle, un radical hétéroaryle et un radical cycloalkyle,
- R₅, R₅' et R₅", identiques ou différents sont choisis indépendamment les uns des autres parmi un radical alkyle éventuellement substitué par un radical aryle ou hétéroaryle, un radical aryle, un radical hétéroaryle et un radical cycloalkyle,
- A représente un radical mono- ou bi-cyclique saturé ou insaturé comportant un total de 5 à 10 atomes (parmi lesquels, au total, 1, 2 ou 3 d'entre eux peuvent éventuellement représenter un hétéroatome choisi parmi l'oxygène, le soufre et/ou l'azote) et éventuellement substitué par un ou plusieurs atomes d'halogène, radicaux alkyle et/ou alkoxy, et
- Hal représente un atome d'halogène,
- ou bien les radicaux -Alk-Y forment ensemble un radical choisi parmi : 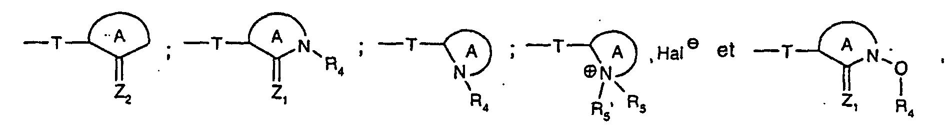 dans lesquels A, R₄, R₅, R₅', Z₁, Z₂ et Hal sont tels que définis précédemment, et T représente soit une liaison soit un radical alkylène linéaire ou ramifié comportant de 1 à 6 atomes de carbone,
à la condition que lorsque R₂ représente un radical hydrocarboné linéaire ou ramifié tel que défini plus haut, Y soit différent du radical 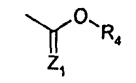 étant entendu que sauf précisions contraires,
- le terme "alkyle" représente un radical alkyle contenant de 1 à 6 atomes de carbone en chaîne droite ou ramifiée et éventuellement substitué par un ou plusieurs atomes d'halogène, radicaux hydroxy et/ou alkoxy,
- le terme "alkoxy" représente un radical alkoxy contenant de 1 à 6 atomes de carbone en chaîne droite ou ramifiée et éventuellement substitué par un ou plusieurs atomes d'halogène et/ou radicaux hydroxy,
- le terme "aryle" représente un radical phényle ou naphtyle, éventuellement substitué par un ou plusieurs atomes d'halogène, radicaux alkyle, hydroxy et/ou alkoxy,
- le terme "hétéroaryle" représente un radical choisi parmi furyle, thiényle, thiazolyle, isothiazolyle, oxazolyle, isoxazolyle, pyrrolyle, imidazolyle, pyrazolyle, pyridyle, pyrazinyle, pyrimidyle, pyridazinyle, indolyle, quinolyle et isoquinolyle, quinazolinyle, éventuellement substitué par un ou plusieurs atomes d'halogène, radicaux alkyle, hydroxy et/ou alkoxy,
- le terme "atome d'halogène" représente un atome de fluor, de chlore, de brome ou d'iode,
- le terme "cycloalkyle représente un radical hydrocarboné cyclique et saturé contenant de 3 à 8 atomes de carbone et éventuellement substitué par un ou plusieurs atomes d'halogène, et/ou radicaux alkyle, hydroxy et/ou alkoxy,
leurs éventuels isomères optiques et/ou géométriques sous forme pure ou en mélange, et leurs éventuels sels d'addition à un acide ou à une base, pharmaceutiquement acceptables.

2. Composés selon la revendication 1 pour lesquels X représente un atome d'oxygène, leurs éventuels isomères optiques et/ou géométriques sous forme pure ou en mélange, ainsi que leurs éventuels sels d'addition à un acide ou à une base, pharmaceutiquement acceptables.

3. Composés selon la revendication 1 pour lesquels R₂ représente le radical cyclopentyle, leurs éventuels isomères optiques et/ou géométriques sous forme pure ou en mélange, ainsi que leurs éventuels sels d'addition à un acide ou à une base, pharmaceutiquement acceptables.

4. Composé selon la revendication 1 qui est la 7-O-(2-oxopropyl)-3'-O-cyclopentyldiosmétine.

5. Composé selon la revendication 1 qui est la 7-O-éthoxycarbonylméthyl-3'-O-cyclopentyldiosmétine.

6. Composé selon la revendication 1 qui est la 7-O-(N,N-diméthylaminocarbonylméthyl)-3'-O-cyclopentyldiosmétine.

7. Composé selon la revendication 1 qui est la 7-O-(N-méthoxy-N-méthylaminocarbonylméthyl)-3'-O-cyclopentyldiosmétine.

8. Composé selon la revendication 1 qui est la 7-O-(N-méthylaminocarbonylméthyl)-3'-O-cyclopentyldiosmétine.

9. Composé selon la revendication 1 qui est la 7-O-(N,N-diméthylaminocarbonylméthyl)-3'-O-(exo-norborn-2-yl)diosmétine.

10. Procédé de préparation des composés selon la revendication 1 **caractérisé en ce que** l'on fait réagir, le composé de formule (II) : 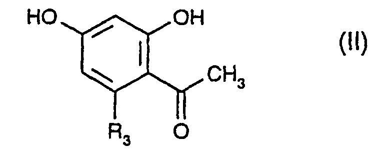 dans laquelle R₃ est tel que défini précédemment,
avec le composé de formule (III) : 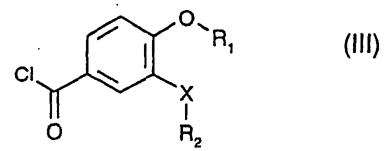 dans laquelle R₁, R₂ et X sont tels que définis précédemment,
en présence d'un excès de bis(triméthylsilyl)amidure de lithium afin d'obtenir le composé de formule (IV) : 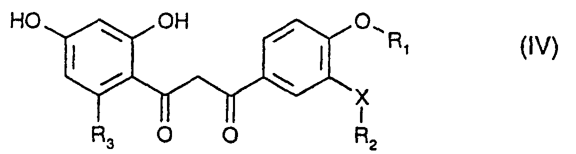 dans laquelle R₁, R₂, R₃ et X sont tels que définis précédemment,
qui est cyclisé, par chauffage dans de l'acide acétique glacial et en présence d'acide sulfurique, en composé de formule (V) : 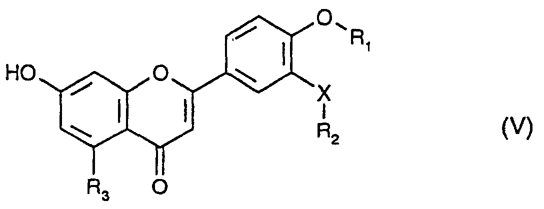 dans laquelle R₁, R₂, R₃ et X sont tels que définis précédemment,
qui est finalement couplé :
- soit avec un composé de formule (VIa) :
Y-Alk-Hal (VIa)
dans laquelle Y et Alk sont tels que définis précédemment et Hal représente un atome d'halogène choisi parmi chlore, brome et iode,
en présence d'hydrogénocarbonate de potassium à chaud, sous atmosphère inerte et en solvant polaire aprotique,
- soit avec un composé de formule (VIb) :
Y-Alk-OH (VIb)
dans laquelle Y et Alk sont tels que définis précédemment,
en présence d'azodicarboxylate d'éthyle et de triphénylphosphine,
afin de conduire aux composés de formule (I) qui peuvent être, le cas échéant et si on le désire :
- séparés suivant une ou plusieurs méthodes de purification choisies parmi la cristallisation, la chromatographie sur gel de silice, l'extraction, la filtration et le passage sur charbon ou résine,
- séparés, sous forme pure ou sous forme de mélange, en leurs éventuels isomères optiques et/ou géométriques, selon des techniques classiques de séparation, et/ou
- transformés, par un acide ou une base, en sels pharmaceutiquement acceptables.

11. Compositions pharmaceutiques contenant les produits de formule (I) selon l'une des revendications 1 à 9 en combinaison avec un ou plusieurs excipients inertes et pharmaceutiquement acceptables.

12. Compositions pharmaceutiques selon la revendication 11 agissant comme inhibiteur de phosphodiestérase du groupe 4.

## Patentansprüche

1. Verbindungen der allgemeinen Formel (I): 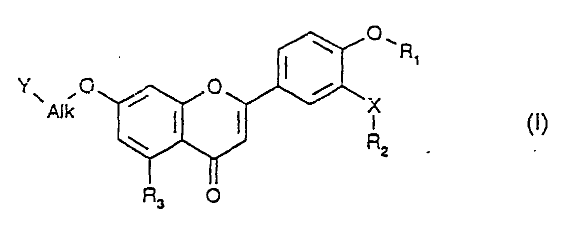 in der:
- R₁ eine Alkylgruppe darstellt,
- R₂ ausgewählt ist aus:
- einer cyclischen Kohlenwasserstoffgruppe, die 3 bis 9 Kohlenstoffatome und gegebenenfalls eine oder mehrere intracyclische Doppelbindungen enthält und die gegebenenfalls durch ein oder mehrere Halogenatome, Alkylgruppen, Alkoxygruppen und/oder Hydroxygruppen substituiert ist,
- eine polycyclische Kohlenwasserstoffgruppe, die 6 bis 15 Kohlenstoffatome enthält, gegebenenfalls eine oder mehrere Doppelbindungen enthält und gegebenenfalls durch ein oder mehrere Halogenatome, Alkylgruppen, Alkoxygruppen und/oder Hydroxygruppen substituiert ist,
- eine geradkettige oder verzweigte Kohlenwasserstoffgruppe, die 1 bis 13 Kohlenstoffatome enthält, gegebenenfalls eine oder mehrere Unsättigungen in Form von Doppelbindungen und/oder Dreifachbindungen aufweist und gegebenenfalls durch ein oder mehrere Halogenatome, Hydroxygruppen, Alkoxygruppen, Arylgruppen, Heteroarylgruppen, cyclische Kohlenwasserstoffgruppen, wie sie oben definiert worden sind, und/oder polycyclische Kohlenwasserstoffgruppen, wie sie oben definiert worden sind, substituiert ist,
- R₃ aus Wasserstoff und der Hydroxygruppe ausgewählt ist,
- Alk eine geradkettige oder verzweigte Alkylengruppe mit 1 bis 6 Kohlenstoffatomen darstellt,
- X aus Sauerstoff, dem Rest -CR'= und dem Rest -CHR'- ausgewählt ist,
- R' aus Wasserstoff und einer Alkylgruppe ausgewählt ist, und
- Y ausgewählt ist aus den Resten: 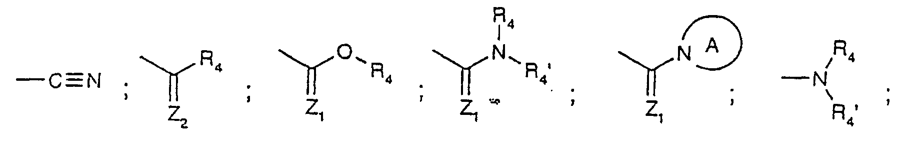 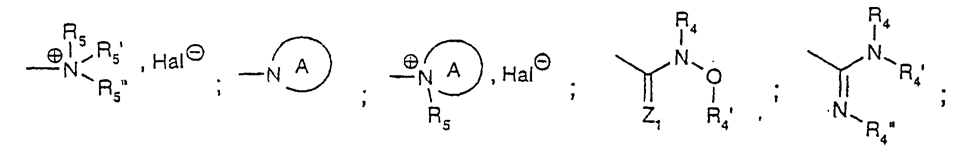 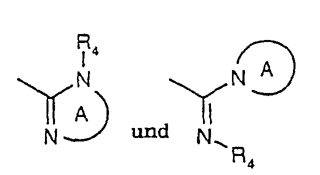 in denen:
- Z₁ Sauerstoff oder Schwefel bedeutet,
- Z₂ Sauerstoff, Schwefel, einen Rest =N-OR₄ oder einen Rest 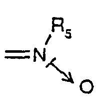 bedeutet,
- R₄, R'₄ und R"₄, die gleichartig oder verschieden sind, unabhängig voneinander aus Wasserstoff, einer gegebenenfalls durch eine Arylgruppe oder eine Heteroarylgruppe substituierten Alkylgruppe, einer Arylgruppe, einer Heteroarylgruppe und einer Cycloalkylgruppe ausgewählt sind,
- R₅, R'₅ und R"₅, die gleichartig oder verschieden sind, unabhängig voneinander aus einer gegebenenfalls durch eine Arylgruppe oder eine Heteroarylgruppe substituierten Alkylgruppe, einer Arylgruppe, einer Heteroarylgruppe und einer Cycloalkylgruppe ausgewählt sind,
- A eine mono- oder bicyclische, gesättigte oder ungesättigte Gruppe, die insgesamt 5 bis 10 Atome aufweist (von denen insgesamt 1, 2 oder 3 gegebenenfalls ein Heteroatom ausgewählt aus Sauerstoff, Schwefel und/oder Stickstoff darstellen können) und gegebenenfalls durch ein oder mehrere Halogenatome, Alkylgruppen und/oder Alkoxygruppen substituiert ist, bedeutet, und
- Hal ein Halogenatom bedeutet,
- oder die Reste -Alk-Y gemeinsam einen Rest bilden ausgewählt aus: 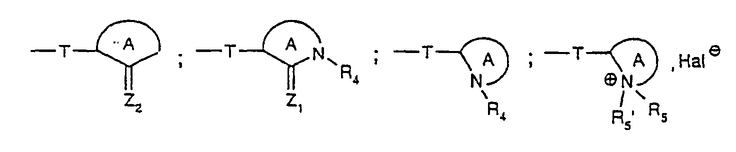 und 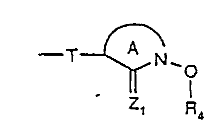 in denen A, R₄, R₅, R₅', Z₁, Z₂ und Hal die oben angegebenen Bedeutungen besitzen und T entweder eine Bindung oder eine geradkettige oder verzweigte Alkylengruppe mit 1 bis 6 Kohlenstoffatomen darstellt,
mit der Maßgabe, wenn R₂ eine geradkettige oder verzweigte Kohlenwasserstoffgruppe. wie sie oben definiert worden ist, darstellt, Y verschieden ist von dem Rest 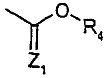
wobei es sich versteht, daß, wenn nichts anderes angegeben ist,
- der Begriff "Alkyl" für eine Alkylgruppe steht, die 1 bis 6 Kohlenstoffatome in gerader oder verzweigter Kette enthält und gegebenenfalls durch ein oder mehrere Halogenatome, Hydroxygruppen und/oder Alkoxygruppen substituiert ist,
- der Begriff "Alkoxy" für eine Alkoxygruppe steht, die 1 bis 6 Kohlenstoffatome in gerader oder verzweigter Kette enthält und gegebenenfalls durch ein oder mehrere Halogenatome und/oder Hydroxygruppen substituiert ist,
- der Begriff "Aryl" für eine Phenyl- oder Naphthylgruppe steht, die gegebenenfalls durch ein oder mehrere Halogenatome, Alkylgruppen, Hydroxygruppen und/oder Alkoxygruppen substituiert ist,
- der Begriff "Heteroaryl" für eine Gruppe steht ausgewählt aus Furyl, Thienyl, Thiazolyl, Isothiazolyl, Oxazolyl, Isoxazolyl, Pyrrolyl, Imidazolyl, Pyrazolyl, Pyridyl, Pyrazinyl, Pyrimidyl, Pyridazinyl, Indolyl, Chinolyl, Isochinolyl und Chinazolinyl, die gegebenenfalls durch ein oder mehrere Halogenatome, Alkylgruppen, Hydroxygruppen und/oder Alkoxygruppen substituiert ist,
- der Begriff "Halogenatom" für ein Fluor-, Chlor-, Brom- oder Iodatom steht,
- der Begriff "Cycloalkyl" für eine cyclische und gesättigte Kohlenwasserstoffgruppe steht, die 3 bis 8 Kohlenstoffatome enthält und gegebenenfalls durch ein oder mehrere Halogenatome und/oder Alkylgruppen, Hydroxygruppen und/oder Alkoxygruppen substituiert ist,
deren eventuelle optische und/oder geometrische Isomeren in reiner Form oder in Form einer Mischung und deren eventuelle pharmazeutisch annehmbaren Additionssalze mit einer Säure oder Base.

2. Verbindungen nach Anspruch 1, worin X ein Sauerstoffatom darstellt, deren eventuelle optische und/oder geometrische Isomere in reiner Form oder in Form einer Mischung sowie deren eventuelle pharmazeutisch annehmbaren Additionssalze mit einer Säure oder Base.

3. Verbindungen nach Anspruch 1, worin R₂ eine Cyclopentylgruppe darstellt, deren eventuelle optische und/oder geometrische Isomere in reiner Form oder in Form einer Mischung sowie deren eventuelle pharmazeutisch annehmbaren Additionssalze mit einer Säure oder Base.

4. Verbindung nach Anspruch 1, nämlich 7-O-(2-Oxopropyl)-3'-O-cyclopentyldiosmetin.

5. Verbindung nach Anspruch 1, nämlich 7-O-Ethoxycarbonylmethyl-3'-O-cyclopentyldiosmetin.

6. Verbindung nach Anspruch 1, nämlich 7-O-(N,N-Dimethylaminocarbonylmethyl)-3'-O-cyclopentyldiosmetin.

7. Verbindung nach Anspruch 1, nämlich 7-O-(N-Methoxy-N-methylaminocarbonylmethyl)-3'-O-cyclopentyldiosmetin.

8. Verbindung nach Anspruch 1, nämlich 7-O-(N,N-Methylaminocarbonylmethyl)-3'-O-cyclopentyldiosmetin.

9. Verbindung nach Anspruch 1, nämlich 7-O-(N,N-Dimethylaminocarbonylmethyl)-3'-O-(exo-norborn-2-yl)-diosmetin.

10. Verfahren zur Herstellung der Verbindungen nach Anspruch 1, **dadurch gekennzeichnet, daß** man die Verbindung der Formel (II): 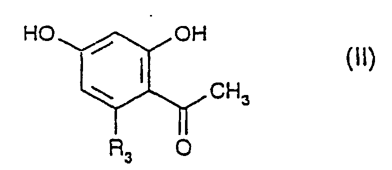 in der R₃ die oben angegebenen Bedeutungen besitzt,
mit der Verbindung der Formel (III): 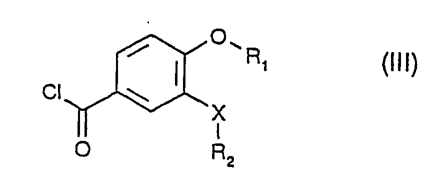 in der R₁, R₂ und X die oben angegebenen Bedeutungen besitzen,
in Gegenwart von Lithium-bis(trimethylsilyl)-amid umsetzt zur Bildung der Verbindung der Formel (IV): 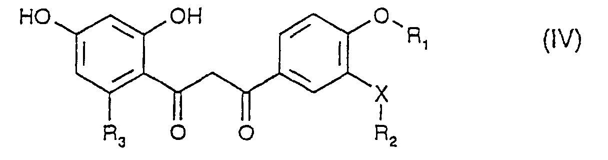 in der R₁, R₂, R₃ und X die oben angegebenen Bedeutungen besitzen,
welche durch Erhitzen in Eisessig und in Gegenwart von Schwefelsäure zu der Verbindung der Formel (V) cyclisiert wird: 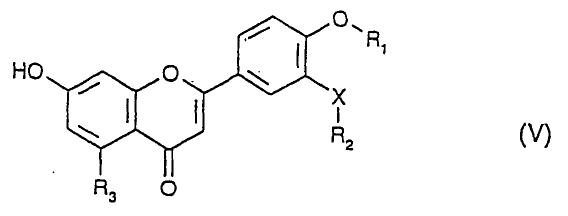 in der R₁, R₂, R₃ und X die oben angegebenen Bedeutungen besitzen, welche schließlich:
- entweder mit einer Verbindung der Formel (VIa):
Y - Alk - Hal (VIa)
in der Y und Alk die oben angegebenen Bedeutungen besitzen und Hal
ein Halogenatom ausgewählt aus Chlor, Brom und Iod bedeutet, in Gegenwart von Kaliumhydrogencarbonat in der Wärme und unter einer inerten Atmosphäre in einem polaren aprotischen Lösungsmittel,
- oder mit einer Verbindung der Formel (VIb):
Y - Alk - OH (VIb)
in der Y und Alk die oben angegebenen Bedeutungen besitzen,
in Gegenwart von Azodicarbonsäureethylester und Triphenylphosphin gekuppelt wird zur Bildung der Verbindungen der Formel (I), welche gegebenenfalls und gewünschtenfalls:
- mit Hilfe einer oder mehrerer Reinigungsmethoden ausgewählt aus Kristallisation, Chromatographie über Kieselgel, Extraktion, Filtration und Überführung über Kohle oder Harz gereinigt werden können,
- mit Hilfe klassischer Trennmethoden in ihre eventuellen optischen und/oder geometrischen Isomeren in reiner Form oder in Form einer Mischung getrennt werden können und/oder
- mit Hilfe einer Säure oder Base in die pharmazeutisch annehmbaren Salze umgewandelt werden können.

11. Pharmazeutische Zubereitungen enthaltend die Produkte der Formel (I) nach einem der Ansprüche 1 bis 9 in Kombination mit einem oder mehreren inerten und pharmazeutisch annehmbaren Trägermaterialien.

12. Pharmazeutische Zubereitungen nach Anspruch 11, die als Inhibitoren der Phosphodiesterase der Gruppe 4 wirken.

## Claims

1. Compounds of general formula (I): 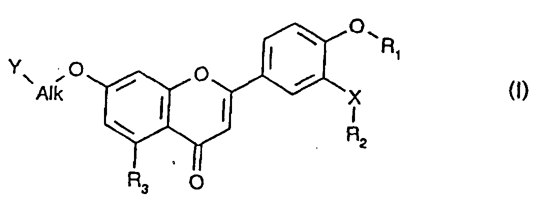 wherein
- R₁ represents an alkyl radical,
- R₂ is selected from:
- a cyclic hydrocarbon radical containing from 3 to 9 carbon atoms, optionally containing one or more intracyclic double bonds, and optionally substituted by one or more halogen atoms, alkyl radicals, alkoxy radicals and/or hydroxy radicals,
- a polycyclic hydrocarbon radical containing from 6 to 15 carbon atoms, optionally containing one or more double bonds and optionally substituted by one or more halogen atoms, alkyl radicals, alkoxy radicals and/or hydroxy radicals,
- a linear or branched hydrocarbon radical containing from 1 to 13 carbon atoms, optionally containing one or more unsaturations in the form of double and/or triple bonds and optionally substituted by one or more halogen atoms, hydroxy radicals, alkoxy radicals, aryl radicals, heteroaryl radicals, cyclic hydrocarbon radicals defined above and/or polycyclic hydrocarbon radicals defined above,
- R₃ is selected from hydrogen and the hydroxy radical,
- Alk represents a linear or branched alkylene radical containing from 1 to 6 carbon atoms,
- X is selected from oxygen, the radical -CR'= and the radical -CHR'-,
- R' is selected from hydrogen and an alkyl radical, and
- Y is selected from the radicals: 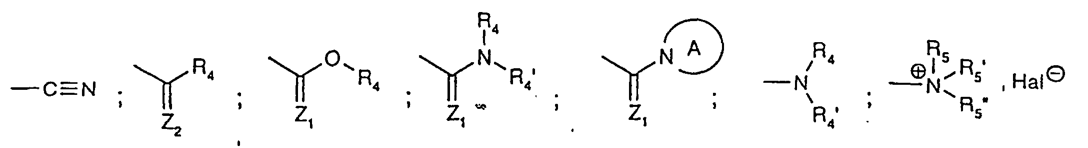 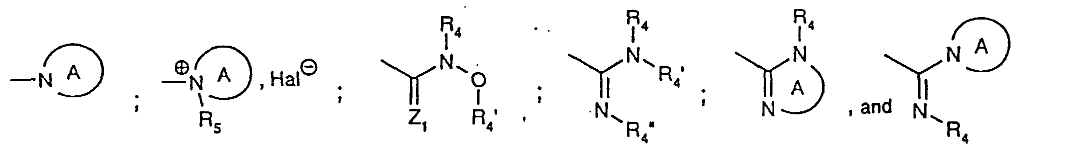 wherein:
- Z₁ represents oxygen or sulphur,
- Z₂ represents oxygen, sulphur, a radical =N-OR₄ or a radical 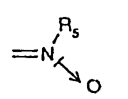
- R₄, R₄' and R₄", which may be identical or different, are selected each independently of the others from hydrogen; an alkyl radical optionally substituted by an aryl or heteroaryl radical; an aryl radical; a heteroaryl radical and a cycloalkyl radical,
- R₅, R₅' and R₅", which may be identical or different, are selected each independently of the others from an alkyl radical optionally substituted by an aryl or heteroaryl radical; an aryl radical; a heteroaryl radical and a cycloalkyl radical,
- A represents a saturated or unsaturated mono- or bi-cyclic radical containing a total of from 5 to 10 atoms (of which a total of 1, 2 or 3 of the atoms may optionally represent a hetero atom selected from oxygen, sulphur and/or nitrogen) and optionally substituted by one or more halogen atoms, alkyl radicals and/or alkoxy radicals, and
- Hal represents a halogen atom,
- or the radicals -Alk-Y together form a radical selected from: 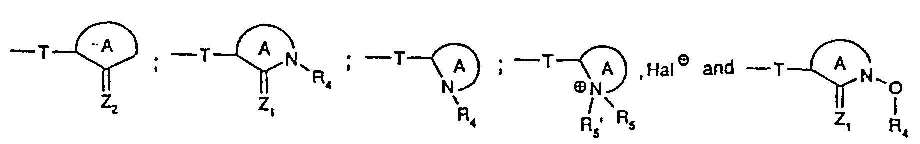
wherein A, R₄, R₅, R₅', Z₁, Z₂ and Hal are as defined above, and T represents either a bond or a linear or branched alkylene radical containing from 1 to 6 carbon atoms,
with the proviso that when R₂ represents a linear or branched hydrocarbon radical as defined above, Y is other than the radical 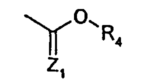 it being understood, unless otherwise specified, that:
- the term "alkyl" represents an alkyl radical containing from 1 to 6 carbon atoms in a straight or branched chain and optionally substituted by one or more halogen atoms, hydroxy radicals and/or alkoxy radicals,
- the term "alkoxy" represents an alkoxy radical containing from 1 to 6 carbon atoms in a straight or branched chain and optionally substituted by one or more halogen atoms and/or hydroxy radicals,
- the term "aryl" represents a phenyl or naphthyl radical, optionally substituted by one or more halogen atoms, alkyl radicals, hydroxy radicals and/or alkoxy radicals,
- the term "heteroaryl" represents a radical selected from furyl, thienyl, thiazolyl, isothiazolyl, oxazolyl, isoxazolyl, pyrrolyl, imidazolyl, pyrazolyl, pyridyl, pyrazinyl, pyrimidyl, pyridazinyl, indolyl, quinolyl and isoquinolyl, quinazolinyl, optionally substituted by one or more halogen atoms, alkyl radicals, hydroxy radicals and/or alkoxy radicals,
- the term "halogen atom" represents a fluorine, chlorine, bromine or iodine atom,
- the term "cycloalkyl" represents a saturated cyclic hydrocarbon radical containing from 3 to 8 carbon atoms and optionally substituted by one or more halogen atoms and/or alkyl, hydroxy and/or alkoxy radicals,
their possible optical and/or geometric isomers in pure form or in the form of a mixture, and possible pharmaceutically acceptable addition salts thereof with an acid or a base.

2. Compounds according to claim 1 wherein X represents an oxygen atom, their possible optical and/or geometric isomers in pure form or in the form of a mixture, and possible pharmaceutically acceptable addition salts thereof with an acid or a base.

3. Compounds according to claim 1 wherein R₂ represents the cyclopentyl radical, their possible optical and/or geometric isomers in pure form or in the form of a mixture, and possible pharmaceutically acceptable addition salts thereof with an acid or a base.

4. Compound according to claim 1 which is 7-O-(2-oxopropyl)-3'-O-cyclopentyldiosmetin.

5. Compound according to claim 1 which is 7-O-ethoxycarbonylmethyl-3'-O-cyclopentyldiosmetin.

6. Compound according to claim 1 which is 7-O-(N,N-dimethylaminocarbonylmethyl)-3'-O-cyclopentyldiosmetin.

7. Compound according to claim 1 which is 7-O-(N-methoxy-N-methylaminocarbonylmethyl)-3'-O-cyclopentyldiosmetin.

8. Compound according to claim 1 which is 7-O-(N-methylaminocarbonylmethyl)-3'-O-cyclopentyldiosmetin.

9. Compound according to claim 1 which is 7-O-(N,N-dimethylaminocarbonylmethyl)-3'-O-(exo-norborn-2-yl)diosmetin.

10. Process for the preparation of compounds according to claim 1, **characterised in that** a compound of formula (II): 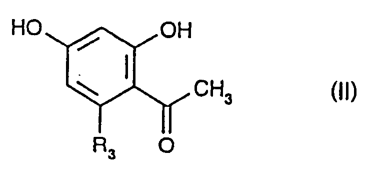 wherein R₃ is as defined hereinbefore,
is reacted with a compound of formula (III): 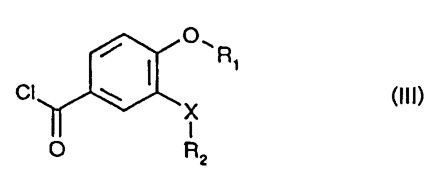 wherein R₁, R₂ and X are as defined hereinbefore,
in the presence of an excess of lithium bis(trimethylsilyl)amide in order to obtain a compound of formula (IV): 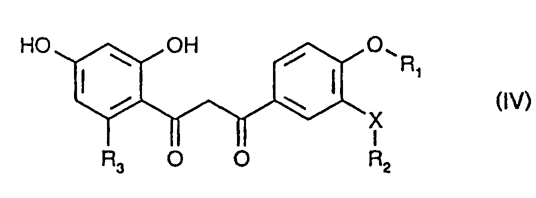 wherein R₁, R₂, R₃ and X are as defined hereinbefore,
which is cyclised, by heating in glacial acetic acid and in the presence of sulphuric acid, to form a compound of formula (V): 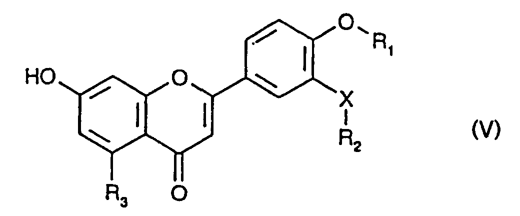 wherein R₁, R₂, R₃ and X are as defined hereinbefore,
which is finally coupled:
- either with a compound of formula (VIa):
Y-Alk-Hal (VIa)
wherein Y and Alk are as defined hereinbefore and Hal represents a halogen atom selected from chlorine, bromine and iodine,
in the presence of hot potassium hydrogen carbonate, under an inert atmosphere and in an aprotic polar solvent,
- or with a compound of formula (VIb):
Y-Alk-OH (VIb)
wherein Y and Alk are as defined hereinbefore,
in the presence of ethyl azodicarboxylate and triphenylphosphine,
in order to yield compounds of formula (I) which may, where appropriate and if desired, be:
- separated in accordance with one or more methods of purification selected from crystallisation, chromatography over silica gel, extraction, filtration and passage over carbon or resin,
- separated, in pure form or in the form of a mixture, into their possible optical and/or geometric isomers, in accordance with conventional separation techniques, and/or
- converted, by an acid or a base, into pharmaceutically acceptable salts.

11. Pharmaceutical compositions comprising the compounds of formula (I) according to any one of claims 1 to 9 in combination with one or more inert pharmaceutically acceptable excipients.

12. Pharmaceutical compositions according to claim 11 that act as group 4 phosphodiesterase inhibitors.
